(19) Europäisches Patentamt
European Patent Office
Office européen des brevets

(11) Veröffentlichungsnummer: **0 226 152**
**B1**

(12) # EUROPÄISCHE PATENTSCHRIFT

(45) Veröffentlichungstag der Patentschrift: **18.01.89**

(51) Int. Cl.⁴: **C 07 C 15/16**, C 07 C 25/18, C 07 C 79/10, C 07 C 6/12, C 07 C 17/33, C 07 C 76/02

(21) Anmeldenummer: **86116981.1**

(22) Anmeldetag: **06.12.86**

(54) **Verfahren zur Herstellung von gegebenenfalls substituierten 2-Benzyl-toluolen.**

(30) Priorität: **18.12.85 DE 3544733**

(43) Veröffentlichungstag der Anmeldung: **24.06.87 Patentblatt 87/26**

(45) Bekanntmachung des Hinweises auf die Patenterteilung: **18.01.89 Patentblatt 89/3**

(84) Benannte Vertragsstaaten: **CH DE FR GB IT LI**

(73) Patentinhaber: **BAYER AG, Konzernverwaltung RP Patentabteilung, D-5090 Leverkusen 1 Bayerwerk (DE)**

(72) Erfinder: **Blank, Heinz Ulrich, Dr., Am Geusfelde 35, D-5068 Odenthal (DE)**
Erfinder: **Silber, Gunter, Dr., Gerstenkamp 21, D-5000 Köln 80 (DE)**

(56) Entgegenhaltungen:
**DE-A- 2 840 272**

**THE JOURNAL OF ORGANIC CHEMISTRY, Band 43, 1978, M. TASHIRO et al. "Studies on selective preparation of aromatic compounds. 15. The Lewis acid catalyzed transalkylation of some tert-butyl-diphenylmethanes and -ethanes in aromatic solvents", pp. 1413-1420**
**ORGANIC PREPARATION AND PRCEDURES INTERNATIONAL, Band 10, Februar 1978, V. BÖHMER et al. "The t-butyl group as a possible protective group in the synthesis of oligo(hydroxy-1,3-phenylene)methylenes", pp. 113-121**
**SYNTHESIS, 1979, no. 12, DEcember 1979, M. TASHIRO "Selective synthesis of aromatic compounds using positional protective groups", pp. 921-936**

(56) Entgegenhaltungen: (Fortsetzung)
**HOUBEN WEYL "Methoden der organischen Chemie", 4. Auflage, Band V/2b "Arene,Arine", 1981, GEORG THIEME VERLAG, Stuttgart, pp. 238-254**
**PATENT ABSTRACTS OF JAPAN, unexamined applications, Feld C, Band 9, no. 317, 12. December 1985, THE PATENT OFFICE JAPANESE GOVERNMENT, p. 64 C 319**

**EP 0 226 152 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

**Beschreibung**

Die vorliegende Erfindung betrifft ein Verfahren zur Herstellung von gegebenenfalls substituierten 2-Benzyl-toluolen durch Umsetzung von gegebenenfalls substituierten 2-Benzyl-tert.-alkyl-toluolen mit einem gegebenenfalls substituierten Kohlenwasserstoff in Gegenwart von wasserfreiem Eisen-(III)-halogenid.

Zur Herstellung von 2-Benzyl-toluolen sind mehrere Verfahren bekannt, so beispielsweise die Alkylierung von Toluol mit Benzylchlorid in Gegenwart von Zinkstaub (Chem. Ber. *6* (1873), S. 907 ff.) bzw. in Gegenwart von Berylliumchlorid (Chem. Ber. *72* (1939), S. 1414 ff.). Es entstehen jedoch schwierig trennbare Gemische aus vorwiegend 2- und 3-Benzyl-toluol, die diese Verfahren für eine technische Anwendung ungeeignet machen.

Ein anderes Verfahren besteht in der Alkylierung von aromatischen Kohlenwasserstoffen mit 2-Methyl-benzychlorid in Gegenwart von Schwefelsäure, Phosphorsäure oder $BF_3$ (DE-OS 24 08 529, DE-OS 24 56 747, EP-OS 37 628, DE-OS 23 36 289). Zu einheitlichen Produkten führen jedoch auch diese Verfahren nur dann, wenn 2-Methyl-benzylchlorid entweder mit unsubstituiertem Benzol oder mit symmetrisch 2fach substituierten Benzolen umgesetzt wird. Anders substituierte Benzol-Derivate ergeben dagegen schwierig trennbare Isomerengemische.

Es ist ausserdem bekannt, dass sich in manchen Fällen mit tert.-Alkylgruppen wie der tert.-Butyl- oder der tert.-Amylgruppe einzelne Positionen an aromatischen Systemen gegenüber Angriffen elektrophiler Agentien schützen lassen (Houben-Weyl Bd. 5/2b (1981), S. 238; Synthesis *1979* S. 921). Nach der Ausführung der elektrophilen Reaktion wird die Schutzgruppe durch Transalkylierung auf ein anderes aromatisches System, beispielsweise mit Hilfe von Friedel-Crafts-Katalysatoren, wieder abgespalten.

Diese Verfahrensweise kann jedoch zur Herstellung einheitlich substituierten Diphenylmethane nicht herangezogen werden, da die Spaltung der Benzylbrücke mit der Abspaltung der Schutzgruppen konkurriert (Houben-Weyl loc. cit.). Lediglich im Sonderfall von 2,2'-Dihydroxy-diphenylmethanen gelang die Abspaltung der tert.-Butylschutzgruppe aus den entsprechend substituierten Ausgangsstoffen in Gegenwart des mild reagierenden Aluminiumchlorid/Nitromethan-Komplexes, während unter gleichen Umständen die Schutzgruppen aus 4,4'-Dihydroxy-diphenylmethanen nur unter gleichzeitig ablaufender Transbenzylierung entfernt werden konnten. Auch in Org. Prep. Proc. Int. *10* (1978), S. 113 wird die Sonderstellung von Diphenylmethanen, die in ortho-Stellung zur Methylengruppe Hydroxylsubstituenten tragen, bestätigt. Hierbei gelang die Abspaltung von Schutzgruppen mit 1,25 Äquivalenten $AlCl_3$, bezogen auf die Diphenylmethanverbindung, in Toluol, während die Abspaltung von tert.-Butylschutzgruppen aus Diphenylmethanen ohne Hydroxygruppen in der Regel zu komplexen Reaktionsgemischen führt. Neben dem genannten Aluminiumchlorid/Nitromethan-Komplex ergaben auch andere typische Friedel-Crafts-Katalysatoren, wie beispielsweise $TiCl_4$ oder $SnCl_4$ unerwünschte Nebenprodukte aus der Übertragung von Benzylgruppen. Bei einer Reihe von Diphenylmethanderivaten konnte mit $TiCl_4$ bzw. $SnCl_4$ überhaupt keine Reaktion erzwungen werden. Lediglich aus 4,4'-bis-tert.-Butyl-diphenylmethan konnten die tert.-Butylgruppen selektiv mit Aluminiumchlorid/Nitromethan abgespalten werden (J. Org. Chem., *43* (1978), S. 1413).

Es wurde nun überraschend gefunden, dass man gegebenenfalls substituierte 2-Benzyl-tert.-alkyl-toluole mit einem gegebenenfalls substituierten aromatischen Kohlenwasserstoff in Gegenwart von wasserfreiem Eisen-(III)-halogenid zu gegebenenfalls substituierten 2-Benzyl-toluolen umsetzen kann.

Es wurde demnach ein Verfahren zur Herstellung von 2-Benzyl-toluolen der Formel

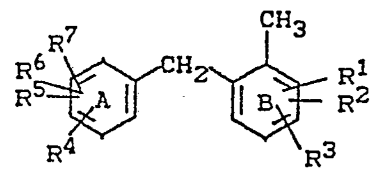

(I)

in der

$R^1$ bis $R^7$ unabhängig voneinander Wasserstoff, Halogen, Alkyl, Aryl, Nitro, Cyano, Acylamino, Aryloxy, Arylsulfonyl oder Alkylsulfonyl bedeuten, wobei im Kern B entweder die para-Position zur Methylgruppe oder die para-Position zur Methylengruppe unsubstituiert ist und wobei je zwei der Reste $R^1$-$R^7$, wenn sie benachbart sind, einen anellierten aromatischen oder cycloaliphatischen Ring bilden können,

gefunden, das dadurch gekennzeichnet ist, dass man 2-Benzyl-tert.-alkyl-toluole der Formel

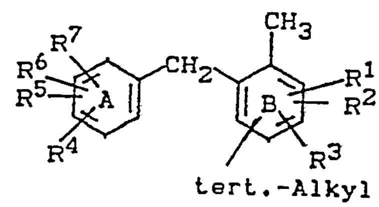

(II)

in der

$R^1$ bis $R^7$ die genannte Bedeutung haben und die tert.-Alkyl-Gruppe in para-Stellung zur Methylgruppe oder in para-Stellung zur Methylengruppe steht,

mit einem Überschuss eines aromatischen Kohlenwasserstoffs der Formel

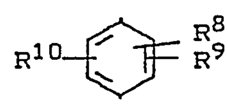

(III)

in der

$R^8$ bis $R^{10}$ unabhängig voneinander Wasserstoff, Alkyl oder Aryl bedeuten, wobei zwei der Reste $R^8$-$R^{10}$, wenn sie benachbart sind, einen anellierten aromatischen oder cycloaliphatischen Ring bilden können,

in Gegenwart von wasserfreiem Fe-(III)-halogenid bei 0-150° C umsetzt.

Als Halogen sei beispielsweise Fluor, Chlor, Brom, Iod, bevorzugt Fluor, Chlor, Brom, besonders bevorzugt Chlor oder Brom genannt.

Als Alkyl sei solches mit 1-10 C-Atomen, bevorzugt 1-6 C-Atomen, besonders 1-4 C-Atomen genannt, wie Methyl, Ethyl, Propyl, Butyl, Hexyl, Octyl, Decyl. In ganz besonders bevorzugter Weise sei Methyl oder Ethyl genannt. Alkyl trägt am α-C-Atom höchstens eine Verzweigung, bevorzugt am α-C-Atom keine Verzweigung und besonders bevorzugt auch an den weiteren C-Atomen keine Verzweigung.

Als Aryl sei beispielsweise Phenyl, Naphthyl, Anthryl, Phenanthryl oder Diphenylyl, bevorzugt Phenyl oder Naphthyl, besonders bevorzugt Phenyl genannt. Solche Arylreste können ihrerseits durch Alkyl oder Halogen substituiert sein.

Zwei der Reste $R^1$ bis $R^7$ können, wenn sie benachbart sind, weiterhin einen anellierten aromatischen oder cycloaliphatischen Ring bilden. Der aromatische Kern A (I) kann einen oder zwei solcher anellierter Ringe tragen. Für den Fall eines anellierten aromatischen Ringes wird aus dem aromatischen Kern A und/oder B beispielsweise das Naphthalin-System, aus dem aromatischen Kern A im Falle der 2fachen Anellierung auch das Anthracen- oder das Phenanthren-System. Für den Fall von anellierten cycloaliphatischen Ringen entstehen in entsprechender Weise das Indan-, das Tetralin-, das Benzo-cycloheptyl- oder das Benzocyclooctyl-System.

Als Acylamino seien beispielsweise Substituenten der Formulierung −NHCO-Alkyl oder −NHCO-Aryl genannt, in denen Alkyl und Aryl die obengenannte Bedeutung haben. In den Aryloxy-, Arylsulfonyl- und Alkylsulfonyl-Substituenten haben Alkyl und Aryl ebenfalls die obengenannte Bedeutung.

Als tertiäre Alkylgruppen seien solche Alkylgruppen genannt, die 4-8 C-Atome haben und in denen das α-C-Atom ein tertiäres ist, beispielsweise tert.-Butyl, tert.-Amyl, tert.-Hexyl oder tert.-Octyl. In bevorzugter Weise sei die tert.-Butyl-Gruppe genannt.

Die tert.-Alkyl-Gruppe steht entweder in para-Stellung zur Methylgruppe oder in para-Stellung zur Methylengruppe.

Im erfindungsgemässen Verfahren werden bevorzugt 2-Benzyl-tert.-alkyl-toluole der Formel

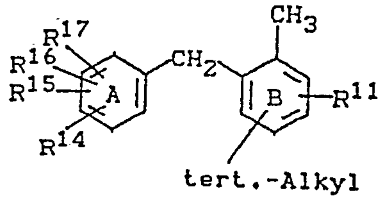

(IV)

eingesetzt, in der

$R^{11}$ und $R^{14}$ bis $R^{17}$ unabhängig voneinander Wasserstoff, Fluor, Chlor, Brom, Nitro, Alkyl oder Phenyl bedeuten, wobei je zwei der Reste $R^{14}$-$R^{17}$, wenn sie benachbart sind, einen anellierten aromatischen oder cycloaliphatischen Ring bilden können und die tert.-Alkyl-Gruppe in para-Stellung zur Methylgruppe oder in para-Stellung zur Methylengruppe steht.

In besonders bevorzugter Weise werden 2-Benzyl-tert.-alkyl-toluole der Formel

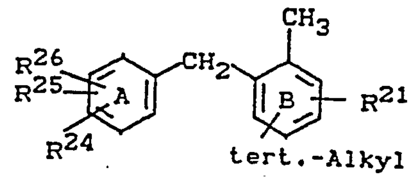

(V)

eingesetzt, in der

$R^{21}$ Wasserstoff oder Alkyl bedeutet,

$R^{24}$-$R^{26}$ den für $R^{14}$ bis $R^{17}$ in (IV) gegebenen Bedeutungsumfang besitzen und die tert.-Alkyl-Gruppe die in (IV) genannte Stellung einnimmt.

In ganz besonders bevorzugter Weise werden 2-Benzyl-tert.-alkyl-toluole der Formel

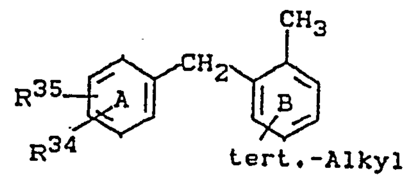

(VI)

eingesetzt, in der

$R^{34}$, $R^{35}$ den für $R^{14}$-$R^{17}$ in (IV) angegebenen Bedeutungsumfang besitzen und die tert.-Alkyl-Gruppe die in (IV) genannte Stellung einnimmt.

Beispiele für im erfindungsgemässen Verfahren einsetzbare 2-Benzyl-tert.-alkyl-toluole sind:

2-Benzyl-4-tert.-butyl-toluol, 2-Benzyl-5-tert.-butyl-toluol, 2-(2-Chlorbenzyl)-4- bzw. -5-tert.-butyl-toluol, 2-(3-Chlorbenzyl)-4- bzw. -5-tert.-butyl-toluol, 2-(4-Chlorbenzyl)-4- bzw. -5-tert.-butyl-toluol, 2-(2,3-Dichlorbenzyl)-4- bzw. -5-tert.-butyl-toluol, 2-(2,4-Dichlorbenzyl)-4- bzw. -5-tert.-butyl-toluol, 2-(2,5-Dichlorbenzyl)-4- bzw. -5-tert.-butyl-toluol, 2-(3,4-Dichlorbenzyl)-4-bzw. -5-tert.-butyl-toluol, 2-(2-Fluorbenzyl)-4- bzw. -5-tert.-butyl-toluol, 2-(3-Fluorbenzyl)-4- bzw. -5-tert.-butyl-toluol, 2-(4-Fluorbenzyl)-4- bzw. -5-tert.-butyl-toluol, 2-(2-Brombenzyl)-4- bzw. -5-tert.-butyl-toluol, 2-(3-Brombenzyl)-4- bzw. -5-tert.-butyl-toluol, 2-(4-Brombenzyl)-4- bzw. -5-tert.-butyl-toluol, 2-(2-Nitrobenzyl)-4- bzw. -5-tert.-butyl-toluol, 2-(3-Nitrobenzyl)-4- bzw. -5-tert.-butyl-toluol, 2-(4-Nitrobenzyl)-4- bzw. -5-tert.-butyl-toluol, 2-(2-Methylbenzyl)-4- bzw. -5-tert.-butyl-toluol, 2-(3-Methylbenzyl)-4- bzw. -5-tert.-butyl-toluol, 2-(4-Methylbenzyl)-4- bzw. -5-tert.-butyl-toluol.

Als aromatischer Kohlenwasserstoff wird erfindungsgemäss in bevorzugter Weise einer der Formel

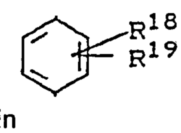

(VII)

eingesetzt, worin

$R^{18}$ und $R^{19}$ den für $R^8$-$R^{10}$ in (III) gegebenen Bedeutungsumfgang haben.

In besonders bevorzugter Weise wird ein aromatischer Kohlenwasserstoff der Formel

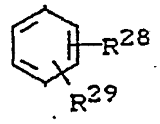

(VIII)

eingesetzt, in der

$R^{28}$ und $R^{29}$ unabhängig voneinander Wasserstoff, Methyl, Ethyl oder Phenyl bedeuten.

Beispiele für aromatische Kohlenwasserstoff für das erfindungsgemässe Verfahren sind: Benzol, Toluol, o-, m-, p-Xylol, 1,2,4-Trimethylbenzol, 1,2,3,4-Tetramethylbenzol, Ethylbenzol, o-, m-, p-Ethyl-toluol, o-, m-, p-Diethylbenzol, n-Propylbenzol, o-, m-, p-(n-Propyl)-toluol, n-Butylbenzol, Tetralin, Naphthalin, 1-, 2-Methyl-naphthalin, 1-, 2-Ethyl-naphthalin, 1-, 2-Phenyl-naphthalin, Diphenyl, 1-, 2-, 3-Methyl-diphenyl. In ganz besonders bevorzugter Weise wird Benzol oder Toluol als aromatischer Kohlenwasserstoff eingesetzt.

Der aromatische Kohlenwasserstoff wird im Überschuss, bezogen auf das 2-Benzyl-tert.-alkyl-toluol eingesetzt, beispielsweise in einer Menge von 1-20 Mol, bevorzugt 2-15 Mol, besonders bevorzugt 4-12 Mol, pro 1 Mol des 2-Benzyl-tert.-alkyl-toluols. Der Einsatz von mehr als 20 Mol des aromatischen Kohlenwasserstoffs ist grundsätzlich möglich, bringt jedoch keine weiteren Vorteile. Selbstverständlich können im erfindungsgemässen Verfahren auch Gemische der genannten aromatischen Kohlenwasserstoffe eingesetzt werden. Darüber hinaus können zusätzliche inerte Lösungsmittel im erfindungsgemässen Verfahren eingesetzt werden. Solche zusätzlichen Lösungsmittel müssen inert gegenüber den übrigen Reaktionsteilnehmern unter den Bedingungen des erfindungsgemässen Verfahrens sein, beispielsweise aliphatische Kohlenwasserstoffe, wie Hexan, Heptan, Octan, Benzinfraktionen, halogenierte aliphatische Kohlenwasserstoffe, wie Methylenchlorid, Tetrachlorethan, halogenierte aromatische Kohlenwasserstoffe mit zwei oder mehr Halogenatomen, wie Dichlorbenzol, Dibrombenzol, Trichlorbenzol, oder Nitrobenzol.

Das erfindungsgemässe Verfahren wird in Gegenwart von wasserfreiem Fe-(III)-halogenid, beispielsweise $FeCl_3$ oder $FeBr_3$ oder einem Gemisch beider, bevorzugt in Gegenwart von $FeCl_3$, durchgeführt. Die Menge des Fe-(III)-halogenids beträgt beispielsweise 0,05-1 Mol, bevorzugt 0,2-0,8 Mol, besonders bevorzugt 0,2-0,6 Mol, pro Mol des 2-Benzyl-tert.-alkyl-toluols. Der Einsatz von mehr als 1 Mol Fe-(III)-halogenid ist grundsätzlich möglich, bringt jedoch keinen weiteren Vorteil.

Das erfindungsgemässe Verfahren wird bei einer Temperatur von 0-150° C, bevorzugt 0-100° C, besonders bevorzugt 5-80° C, durchgeführt. Die Wahl der Temperatur hängt von der eingesetzten Menge an Katalysator ab. So ist es beispielsweise vorteilhaft, bei höheren Temperaturen mehr Katalysator zuzusetzen, um eine weitgehend quantitative Abspaltung der tert. Butylgruppe zu erzielen. Andererseits kann bei niedrigeren Temperaturen in vorteilhafter Weise weniger Katalysator verwendet werden. Es kann bei Normaldruck, Überdruck oder Unterdruck gearbeitet werden. Beispielsweise wird ein Überdruck angelegt, um niedrig siedende Reaktionspartner bei der gewählten Reaktionstemperatur in der flüssigen Phase zu halten. In bevorzugter Weise wird bei Normaldruck gearbeitet.

Die Reihenfolge des Zusammengebens der Reaktionspartner ist grundsätzlich beliebig. Man kann beispielsweise sämtliche Reaktionspartner bei Raumtemperatur vorlegen und das Reaktionsgemisch auf die gewünschte Temperatur bringen. Eine weitere Verfahrensweise besteht darin, das Eisen-(III)-halogenid in der Gesamtmenge des vorgesehenen aromatischen Kohlenwasserstoffes vorzulegen und auf Reaktionstemperatur zu erhitzen und anschliessend das 2-Benzyl-tert.-alkyltoluol zuzudosieren. Alternativ kann auch ein Teil des aromatischen Kohlenwasserstoffs als Lösungsmittel für das 2-Benzyl-tert.-alkyl-toluol dienen.

Die Reaktionszeit ist abhängig von der eingesetzten molaren Menge an Eisen-(III)-halogenid und der gewählten Reaktionstemperatur. Das Reaktionsende lässt sich beispielsweise in einer dem Fachmann geläufigen Weise durch chromatographische Untersuchung von entnommenen Proben des Reaktionsgemisches bestimmen. Eine geringe Nachreaktionszeit kann vor der Aufarbeitung noch angesetzt werden.

Zur Aufarbeitung des Reaktionsgemisches kann beispielsweise das Eisen-(III)-halogenid durch Zusatz von Wasser oder verdünnter Mineralsäure bei 0-100° C hydrolisiert werden. Das anfallende wässerig/organische Gemisch kann vor der Phasentrennung einer Klärfiltration unterworfen werden, falls dies gewünscht wird. Hierzu kann etwa über übliche säurebeständige Filter, gegebenenfalls nach Zusatz von handelsüblichen Klär- und/oder Filtrierhilfsmitteln, wie Cellulosepulvern, Tonsil, Aktivkohle und/oder Kieselgur, filtriert werden. Nach der Phasentrennung wird die organische Phase üblicherweise mit Wasser und/oder 1-10 gew.-%iger Natriumhydrogencarbonatlösung gewaschen. Die weitere Aufarbeitung der organische Phase erfolgt nach üblichen Methoden, beispielsweise durch Destillation oder Kristallisation. Bei der Destillation fällt im allgemeinen als erste Fraktion der Überschuss des eingesetzten aromatischen Kohlenwasserstoffs an; dieser kann nach Trocknung erneut im erfindungsgemässen Verfahren verwendet werden. Als weitere Fraktionen kann man den durch die tert.-Alkyl-Gruppen substituierten aromatischen Kohlenwasserstoff und das erfindungsgemäss angestrebte 2-Benzyltoluol durch Feindestillation, gegebenenfalls im Vakuum, oder durch fraktionierte Kristallisation isolieren.

In einer weiteren Ausgestaltung der Aufarbeitung des Reaktionsgemisches aus dem erfindungsgemässen Verfahren wird dieses Reaktionsgemisch ohne Zusatz von Wasser oder verdünnter Mineralsäure vom ungelösten Eisen-(III)-halogenid abfiltriert. Das Filtrat wird sodann destillativ aufgearbeitet, wobei der zurückgewonnene Überschuss des aromatischen Kohlenwasserstoffs in wasserfreier Form anfällt und ohne weitere Vorbereitung wieder im erfindungsgemässen Verfahren eingesetzt werden kann.

Die im erfindungsgemässen eingesetzten 2-Benzyl-tert.-alkyl-toluole sind bekannte Verbindungen oder können nach bekannten Methoden hergestellt werden (DE-OS 28 40 272; Zh. Org. Khim., *19* (1983), S. 1674 im Original bzw. S. 1484 in der englischen Übersetzung). So kann beispielsweise p-tert.-Butyl-toluol mit gegebenenfalls substituierten Benzylhalogeniden in Gegenwart von Friedel-Crafts-Katalysatoren, wie $AlCl_3$, $FeCl_3$ oder $TiCl_4$ und gegebenenfalls in inerten Lösungsmitteln in an sich bekannter Weise umgesetzt werden.

In einer weiteren Ausgestaltung des erfindungsgemässen Verfahrens wird die Umsetzung eines Benzylhalogenids der Formel

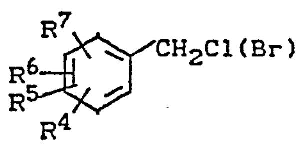

(IX)

in der

$R^4$ bis $R^7$ die obengenannte Bedeutung haben, mit einem tert.-Alkyl-toluol der Formel

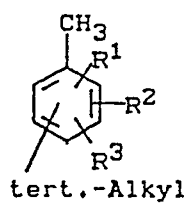

(X)

in der

$R^1$ bis $R^3$ die obengenannte Bedeutung besitzen, eine ortho-Position zur Methylgruppe frei ist und die tert.-Alkylgruppe in para-Position zur Methylgruppe oder zur genannten freien ortho-Position steht,

bei erhöhter Temperatur, beispielsweise 80-200° C, bevorzugt 100-150° C, und in Gegenwart von Eisen-(III)-halogenid als eine erste Stufe im Gesamtverfahren durchgeführt.

(X) wird hierbei im Überschuss, beispielsweise in einer Menge von 2-20 Mol, bevorzugt 5-15 Mol, besonders bevorzugt 5-10 Mol, pro Mol (IX) eingesetzt. Das Eisen-(III)-halogenid wird beispielsweise in einer Menge von 0,01-0,5 Mol, bevorzugt 0,01-0,1 Mol, pro Mol (IX) eingesetzt.

Nach dem Ende der beschriebenen ersten Reaktionsstufe wird das überschüssige (X) abdestilliert und kann ohne weitere Reinigung erneut in diese erste Stufe zurückgeführt werden. Der Rückstand dieser ersten Reaktionsstufe kann in der oben beschriebenen Form erfindungsgemäss mit einem aromatischen Kohlenwasserstoff (III) versetzt werden, eine etwa fehlende Menge an Eisen-(III)-halogenid durch weitere Zugabe von Eisen-(III)-halogenid ergänzt werden und die zweite Stufe im oben beschriebenen Sinne durchgeführt werden.

Grundsätzlich ist es jedoch auch möglich, den Überschuss des tert.-Alkyl-toluols (X) im Reaktionsgemisch aus der beschriebenen ersten Stufe zu belassen und nach Zusatz des aromatischen Kohlenwasserstoffs die zweite Stuffe durchzuführen. Es ist jedoch bevorzugt, den Überschuss an (X) vor Durchführung der zweiten Stufe abzudestillieren.

In einer noch weiteren Ausgestaltung des erfindungsgemässen Verfahrens hat sich gezeigt, dass es vorteilhaft ist, als aromatischen Kohlenwasserstoff ein Toluol der Formel

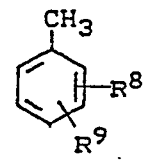

(XI)

einzusetzen, in der

$R^8$ und $R^9$ unabhängig voneinander Wasserstoff, Alkyl oder Aryl bedeuten und für den Fall, dass sie benachbart sind, auch einen anellierten aromatischen oder cycloaliphatischen Ring bilden können und worin eine der ortho-Positionen zur Methylgruppe frei ist und die para-Position zur Methylgruppe oder zur genannten freien ortho-Position ebenfalls frei ist, aus dem im Verlaufe der Reaktion ein tert.-Alkyl-toluol der Formel

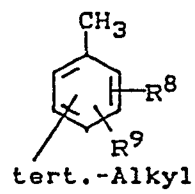

(XII)

entsteht, in der

$R^8$ und $R^9$ die genannte Bedeutung haben und worin eine der ortho-Positionen zur Methylgruppe frei ist und die tert.-Alkyl-Gruppe in para-Position zur Methylgruppe oder zur genannten freien ortho-Position steht.

Dieses substituierte tert.-Alkyl-toluol der Formel (XII) kann sodann, wie oben beschrieben wurde, isoliert werden und mit einem Benzylhalogenid (IX) in der oben beschriebenen ersten Stufe erneut umgesetzt werden.

Bei der weiteren Untersuchung des erfindungsgemässen Verfahrens in seiner zweistufigen Variante, wie weiter oben beschrieben, hat sich gezeigt, dass beim Einsatz substituierter Toluole (XI) zum grössten Teil tert.-Alkyl-toluole (XII) entstehen, in denen die tert.-Alkyl-Gruppe in para-Position zur Methylgruppe steht, sofern die para-Position frei ist. Während der Reaktion und einer evtl. angestezten Nachreaktionszeit isomerisiert jedoch das para-substituierte (XII) zum Teil zum metaisomeren, sofern eine der meta-Positionen zur Methylgruppe frei ist. Es war nun ausserordentlich überraschend, dass auch Gemische der tert.-Alkyl-toluole (XII), in denen die tert.-Alkyl- und die Methyl-Gruppe nebeneinander als para- und meta-ständig vorkommen und die eine freie ortho-Position zur Methyl-gruppe haben, die gleichzeitig zur genannten meta-ständigen tert.-Alkyl-Gruppe in para-Position steht, ohne Einbusse an Selektivität rezyklisiert, d.h. mit einem Benzylhalogenid (IX) zum Ausgangsstoff des erfindungsgemäss einzusetzenden 2-Benzyl-tert.-alkyl-toluols umgesetzt werden können. Dieser Fall ist beispielsweise dann gegeben, wenn der aromatische Kohlenwasserstoff (XI) das unsubstituierte Toluol oder ein Toluol der Formel

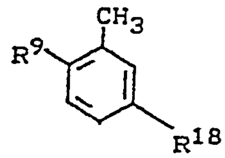

(XIII)

ist, in der

$R^9$ die genannte Bedeutung hat und

$R^{18}$ Alkyl oder Aryl bedeutet.

Das erfindungsgemässe Verfahren ist in seiner glatten und selektiven Durchführbarkeit überraschend und war in keiner Weise bei Kenntnis des Standes der Technik vorhersehbar. Es war nämlich davon auszugehen, dass die Phenylmethanderivate mit einer tert.-Alkyl-Gruppe in Gegenwart eines aromatischen Kohlenwasserstoffs und eines Friedel-Krafts-Katalysators entweder keine Reaktionen eingehen oder unter Transbenzylierung zu einem uneinheitlichen Gemisch führen würden, in dem die gewünschte Verbindung entweder nur in geringen Mengen enthalten ist oder aus dem sie mindestens unter Ausbeuteverlusten und hohen Kosten nur mühsam isoliert werden könnte.

Die erfindungsgemäss herstellbaren 2-Benzyltoluole sind Zwischenprodukte, etwa zur Herstellung von wertvollen Farbstoffen, beispielsweise für den Fall, dass im aromatischen Kern A von (I) eine der ortho-Positionen zur Methylengruppe unsubstituiert ist, zur Herstellung von Anthrachinonfarbstoffen über die zugehörigen Anthracene und Anthrachinone (EP-A 37 628).

*Beispiel 1*

52,8 g (0,2 Mol) 2-Benzyl-4-tert.-butyl-toluol (89,9%ig), 184,3 g (2,0 Mol) trockenes Toluol und 8,11 g (0,05 Mol) Eisen-(III)-chlorid (wasserfrei, sublimiert) wurden in einer Rührapparatur, versehen mit Rührer, Rückflusskühler und Thermometer, vorgelegt. Man erhitzte unter Rühren 6 h auf 50° C, kühlte ab, fügte zum Reaktionsgemisch 250 ml Wasser zu und trennte nach gutem Durchrühren die wässerige von der organischen Phase ab. Die wässerige Phase wurde 1 mal mit 300 ml Methylenchlorid extrahiert, und die organischen Phasen je 1 mal mit 100 ml 10%iger wässeriger Salzsäure und 2 mal mit je 100 ml Wasser gewaschen. Nach Trocknen der vereinigten organischen Phasen über Natriumsulfat wurden die leicht flüchtigen Anteile am Rotationsverdampfer abgezogen. Der Rückstand wog 65,8 g und enthielt nach gaschromatographischer Analyse 47,6% = 31,3 g 2-Benzyltoluol. Die Rohausbeute betrug damit 86% der theoretischen Ausbeute.

Man fraktionierte das Reaktionsgemisch mit Hilfe einer 30-cm-Vigreux-Kolonne im Vakuum. Nach einem Vorlauf von 15,41 g erhielt man bei einer Temperatur von 40-94° C/1,2 mbar 5,8 g einer Zwischenfraktion, die nach gaschromatographischer Analyse zu 52,9% aus 2-Benzyl-toluol bestand. Die Hauptfraktion ging einheitlich bei 95-99° C/1,2 mbar über (24,73 g, nach gaschromatographischer Analyse 97,5%ig). Anschliessend konnte noch ein Nachlauf bei 92-123° C/1,2 mbar gewonnen werden (7,75 g, nach gaschromatographischer Analyse 38,1%ig).

Die destillierte Ausbeute, berechnet über sämtliche Fraktionen betrug 83% der theoretischen Ausbeute, berechnet auf die Hauptfraktion 66% der theoretischen Ausbeute.

Das erhaltene 2-Benzyl-toluol wurde durch kernresonanzspektroskopie, durch Infrarotspektroskopie und durch Massenspektroskopie identifiziert und als identisch mit einer Probe erkannt, die aus 2-Methylbenzylchlorid und Benzol hergestellt worden war.

*Beispiel 2* (Vergleichsbeispiel)

In einem Kölbchen, versehen mit Magnetrührer und Rückflusskühler wurden 2,37 g (9 mmol) 2-Benzyl-4-tert.-butyl-toluol (90%ig) und 9,21 g (100 mmol) Toluol trocken vorgelegt. Man fügte 0,95 g (5 mmol) $TiCl_4$ zu und liess 6 h bei 50° C rühren. Nach gaschromatographischer Analyse fand keine Umsetzung statt.

*Beispiel 3* (Vergleichsbeispiel)

Es wurde wie in Beispiel 2, jedoch bei 100° C gearbeitet. Nach gaschromatographischer Analyse fand keine Umsetzung statt.

*Beispiel 4*

2,37 g (9 mmol) 2-Benzyl-4-tert.-butyl-toluol (90%ig) und 7,81 g (100 mmol) trockenes Benzol wurden in einem Kölbchen mit Magnetrührer und Rückflusskühler vorgelegt. Man fügte 0,811 g trockenes, sublimiertes Eisen-(III)-chlorid zu und liess 6 h bei 50° C rühren. Nach gaschromatographischer Analyse wurde eine Rohausbeute von 82% der theoretischen Ausbeute erziell.

*Beispiele 5-7*

In den Beispielen 5-7 ist die Abhängigkeit der Reaktion von der Katalysatormenge aufgezeigt.

Allgemeine Vorschrift: In einem Kölbchen wurden 2,37 g (9 mmol) 2-Benzyl-4-tert.-butyltoluol (90%ig) und 9,21 g (100 mmol) trockenes Toluol eingewogen. Man fügte Eisen-(III)-chlorid zu und liess 6 h bei 50° C rühren. Dann wurde das Reaktionsgemisch gaschromatographisch untersucht. Die Ergebnisse sind in Tabelle 1 aufgelistet.

*Tabelle 1*

| Eisen-(III)-chlorid | | Ausbeute | Ausbeute, bezogen auf Umsatz |
|---|---|---|---|
| [g] | [mmol] | [% d. Th.] | [% d. Th.] |
| 0,162 | 1 | 39 | 89 |
| 0,324 | 2 | 92 | 99 |
| 0,811 | 5 | 82 | 91 |

*Beispiel 8*

In einer Apparatur wie in Beispiel 1 wurden 111,05 g (0,36 Mol) 2-(2'-Chlor-benzyl)-4-tert.-butyl-toluol (89%ig), 334,5 g (3,63 Mol) trockenes Toluol und 29,44 g (0,18 Mol) wasserfreies, sublimiertes Eisen-(III)-chlorid vorgelegt. Man liess 1,5 h bei 50° C rühren, kühlte auf Raumtemperatur ab, filtrierte und zog die leicht flüchtigen Anteile am Rotationsverdampfer ab. Der Rückstand (143,5 g) wurde einer fraktionierten Destillation im Vakuum über eine 30-cm-Vigreux-Kolonne unterworfen.

Nach einem Vorlauf von 37,77 g konnte eine Zwischenfraktion bei 42-106° C/0,4 mbar gewonnen werden (5,78 g; nach gaschromatographischer Analyse 26,4%ig). Der Hauptlauf ging einheitlich bei 106-106,5° C/0,4 mbar über (72,76 g, nach gaschromatographischer Analyse 94,9%ig). Als Nachlauf erhielt man bei 106,5-130° C/0,4 mbar noch weitere 6,9 g Produkt (nach gaschromatographischer Analyse 53,6%ig).

Die destillierte Ausbeute über sämtliche Fraktionen betrug damit 94% der theoretischen Ausbeute; berechnet auf die Hauptfraktion, konnten 88% der theoretischen Ausbeute isoliert werden.

Das erhaltene 2-(2-Chlor-benzyl)-toluol wurde durch Kernresonanzspektroskopie, durch Infrarotspektroskopie und durch Massenspektroskopie identifiziert.

*Beispiel 9* (Vergleichsbeispiel)

In einem Kölbchen, versehen mit Magnetrührer und Rückflusskühler, wurden 3,02 g (0,01 Mol) 2-(2-Chlorbenzyl)-4-tert.-butyl-toluol (90,4%ig) und 7,81 g (0,1 Mol) trockenes Benzol vorgelegt. Man fügte 0,7 g (0,005 Mol) $AlCl_3$ (95%ig) zu und liess 6 h bei 50° C rühren. Das Produktgemisch wurde über Gaschromatographie und Massenspektroskopie analysiert. Es wurden folgende Produkte erhalten:

| | |
|---|---|
| Benzol | 70,1% |
| Toluol | 8,5% |
| tert.-Butyl-benzol | 6,5% |
| Diphenyl-methan | 7,4% |
| 2-Benzyl-1-chlor-benzol | 1,3% |
| 2-(2-Chlorbenzyl)-toluol | 0,4% |

sowie weitere, nicht näher identifizierte Verbindungen in kleiner Menge.

*Beispiel 10*

In einer Apparatur wie in Beispiel 1 wurden 300,6 g (2 Mol) p-tert.-Butyl-toluol (98,7%ig) und 1,0 g (0,006 Mol) Eisen(III)chlorid, wasserfrei, sublimiert, vorgelegt und das Gemisch auf 135° C erhitzt. Man dosierte innerhalb von 4 h 33,2 g (0,2 Mol) o-Chlor-benzylchlorid (97%ig) unter Rühren zu und erhöhte die Temperatur anschliessend auf 150° C. Es wurde 6 h bei dieser Temperatur gerührt. Nach Abkühlen wurde das überschüssige p-tert.-Butyl-toluol i-Vak. abdestilliert. Man gewann 267,4 g 99,5%iges tert.-Butyl-toluol-Gemisch, bestehend aus 97,1% p-tert.-Butyl- und 2,9% m-tert.-Butyl-toluol, zurück, entspr. einer Menge von 89% vom Einsatz = 98% der theoretischen Menge.

Der Destillationssumpf wurde mit 184,3 g (2 Mol) trockenem Toluol und 16,2 g (0,1 Mol) wasserfreiem, sublimiertem Eisen-(III)-chlorid versetzt und das Reaktionsgemisch 6 h bei 50° C gerührt.

Dann wurde filtriert und das Reaktionsgemisch einer fraktionierten Destillation über eine 20 cm-Vigreux-Kolonne unterworfen.

Als Vorlauf gingen bei 30° C/20 mbar 154,5 g Toluol (99%ig) über. Als 1. Zwischenlauf konnten bei 31-36° C/0,9 mbar 20,17 g eines 93%igen tert.-Butyl-toluol-Gemisches, bestehend aus 65,1% m-tert.-Butyl- und 34,9% p-tert.-Butyltoluol, isoliert werden.

Der sich anschliessende 2. Zwischenlauf, der bei 62-118° C/1,5 mbar überging, ergab 2,72 g (nach gaschromatographischer Analyse 48,9%ig an 2-(2-Chlor-benzyl)-toluol). Schliesslich konnten bei 118-120° C/1,5 mbar 32,15 g 2-(2-Chlorbenzyl)-toluol (92,8%ig) gewonnen werden. Die destillierte Ausbeute über alle Fraktionen betrug damit 72% der theoretischen Ausbeute, bezogen auf 2-Chlorbenzylchlorid.

*Beispiel 11*

In einem Kölbchen, versehen mit Magnetrührer und Rückflusskühler, wurden 3,49 g (10,00 mMol) eines Isomerengemisches, bestehend aus 45% 2-(2-Chlorbenzyl)-4-tert.-butyl-toluol und 55% -5-tert.-butyltoluol (Gesamtgehalt der beiden Isomeren im Einsatzmaterial: 78%), 9,2 g (100,00 mMol) Toluol, getrocknet, und 0,8 g (5,00 mMol) wasserfreies, sublimiertes Eisen-(III)-chlorid vorgelegt. Man rührte das Gemisch 6 h bei 50° C. Dann wurde auf Raumtemperatur gekühlt und das Gemisch (13,5 g) gaschromatographisch analysiert. Als einziges Diphenylmethanderivat konnten 15,7% 2-(2-Chlorbenzyl)-toluol (entspricht der theoretischen Ausbeute) nachgewiesen werden.

*Beispiel 12*

In einem Kölbchen, versehen mit Magnetrührer, Rückflusskühler und Thermometer, wurden 3,68 g (40,00 mMol) trockenes Toluol, 1,298 g (8,00 mMol) wasserfreies Eisen-(III)-chlorid und 6,04 g (22,02 mMol) eines 99,5%igen Gemisches aus 2-(2-Chlorbenzyl)-tert.-butyl-toluolen vorgelegt. Man liess 6 h bei 50° C rühren. Dann wurde das Reaktionsgemisch auf Eis-Wasser ausgetragen, die organische Phase abgetrennt, die wässerige Phase einmal mit 20 ml Toluol extrahiert, die organischen Phasen mit Wasser und 3%iger wässeriger Natriumhydrogencarbonatlösung gewaschen und über $Na_2SO_4$ getrocknet.

Nach Abtrennen des Lösungsmittels im Rotationsverdampfer verblieben 8,15 g eines bräunlichen Öls, welches nach gaschromatographischer Analyse 36,7%ig an 2-(2-Chlorbenzyl)-toluol war. Die Ausbeute betrug damit 63% der theoretischen Ausbeute, bezogen auf eingesetztes Gemisch.

*Beispiele 13 bis 17*

In den Beispielen 13-17 werden an 2-(2-Chlorbenzyl)-tert.-butyl-toluolgemischen die Abhängigkeit der Reaktion von der Temperatur dargestellt.

*Allgemeine Vorschrift:*

In einem Kölbchen, versehen mit Magnetrührer, Rückflusskühler und Thermometer, wurden 3,02 g (11,01 mMol) eines 99,5%igen Gemisches aus 2-(2-Chlor-benzyl)-tert.-butyl-toluolen, 9,21 g (100,000 mMol) trockenes Toluol und 0,162 g (1,00 mMol) wasserfreies Eisen-(III)-chlorid vorgelegt. Man liess 6 h bei der jeweiligen Temperatur rühren und arbeitete dann wie in Beispiel 12 beschrieben auf. Die Ausbeuten, berechnet nach gaschromatographischer Analyse des aufgearbeiteten Reaktionsgemisches, sind in Tabelle 2 zusammengestellt.

*Tabelle 2*

| Beispiel | Temperatur (° C) | Ausbeute (% d. Th.) |
|---|---|---|
| 13 | 0 | 7 |
| 14 | 24 | 57 |
| 15 | 50 | 46 |
| 16 | 80 | 17 |
| 17 | 110 | 16 |

*Beispiel 18*

In einer Apparatur wie in Beispiel 1 wurden 92,14 g (1,0 Mol) trockenes Toluol, 8,2 g (0,05 Mol) wasserfreies Eisen-(III)-chlorid und 38,3 g (0,11 Mol) eines Gemisches aus isomeren 2-(2,5-Dichlor-benzyl)-tert.-butyl-toluolen, welches zusätzlich noch 5,5% 2-(2,5-Dichlorbenzyl)-toluol enthielt, vorgelegt. Der Ansatz wurde auf 50° C erwärmt und 6 h bei dieser Temperatur gerührt. Dann filtrierte man und destillierte im Vakuum über eine 30-cm-Vigreux-Kolonne das überschüssige Toluol ab. Der Rückstand (47,5 g) wurde im Vakuum über eine 20-cm-Vigreux-Kolonne fraktioniert.

Als Vorlauf gingen bei 23-82° C/0,7 mbar 11,87 g eines Gemisches aus Toluol, 3- und 4-tert.-Butyl-toluol über. Bei 100-125° C/0,6 mbar erhielt man in einer Zwischenfraktion 1,4 g eines farblosen Öls, welches nach gaschromatographischer Analyse 62,6% 2-(2,5-Dichlorbenzyl)-toluol enthielt. Der Hauptlauf destillierte einheitlich bei 127-131° C/0,6 mbar. Es konnten 26,55 g farbloses Öl gewonnen werden, das lt. gaschromatographischer Analyse zu 90,5% aus 2-(2,5-Dichlorbenzyl)-toluol bestand.

Die destillierte Ausbeute über sämtliche Fraktionen errechnet sich wie folgt:

Destillat aus Zwischenfraktion und Hauptlauf 100%iges Produkt: 24,91 g
*minus im Ausgangsmaterial enthaltenes Produkt:* 2,11 g
bei der Reaktion entstandenes Produkt: 22,80 g
100%iges Material

entsprechend 83% der theoretischen Ausbeute, bezogen auf eingesetztes Isomerengemisch.

*Beispiel 19*

In einer Apparatur wie in Beispiel 1 wurden 25,94 g (0,08 Mol) 2-(4-Nitrobenzyl)-4-tert.-butyl-toluol (90%ig, hergestellt durch Umsetzung von p-tert.-Butyl-toluol mit 4-nitro-benzylchlorid in Gegenwart von $FeCl_3$ als Katalysator, entsprechend Beispiel 11, Ausbeute: 46% d. Th. destilliert), 75,91 g (0,82 Mol) trockenes Toluol und 6,68 g (0,04 Mol) wasserfreies, sublimierten Eisen-(III)-chlorid vorgelegt und das Reaktionsgemisch 6 h bei 50° C gerührt. Dann filtrierte man und destillierte im Vakuum über eine 20-cm-Vigreux-Kolonne. Nach einem Vorlauf von 6,44 g wurde bei 80-163° C/1,3 mbar eine Zwischenfraktion gewonnen.

(2,38 g, nach gaschromatographischer Analyse 75,5% 2-(4-Nitro-benzyl)-toluol). Der Hauptlauf (6,85 g, 88,9%ig an Produkt) ging bei 163-167° C/1,3 mbar über.

In einer anschliessenden, bei 167° C/1,3 mbar übergehenden Fraktion wurden weitere 8,25 g Produkt isoliert (lt. gaschromatographischer Analyse 81,2%ig). Die destillierte Ausbeute über sämtliche Fraktionen betrug demnach 14,59 g 100%iges Material (entspricht 80% der theoretischen Ausbeute).

Die erhaltene Verbindung wurde durch spektroskopische Methoden identifiziert.

**Patentansprüche**

1. Verfahren zur Herstellung von 2-Benzyltoluolen der Formel

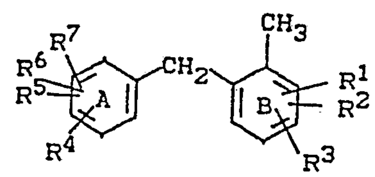

in der

$R^1$ bis $R^7$ unabhängig voneinander Wasserstoff, Halogen, Alkyl, Aryl, Nitro, Cyano, Acylamino, Aryloxy, Arylsulfonyl oder Alkylsulfonyl bedeuten, wobei im Kern B entweder die para-Position zur Methylgruppe oder die para-Position zur Methylengruppe unsubstituiert ist und wobei je zwei der Reste $R^1$-$R^7$, wenn sie benachbart sind, einen anellierten aromatischen oder cycloaliphatischen Ring bilden können,

dadurch gekennzeichnet, dass man 2-Benzyl-tert.-alkyl-toluole der Formel

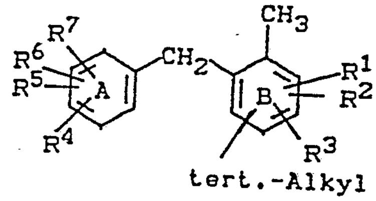

in der

$R^1$ bis $R^7$ die genannte Bedeutung haben und die tert.-Alkyl-Gruppe in para-Stellung zur Methylgruppe oder in para-Stellung zur Methylengruppe steht,
mit einem Überschuss eines aromatischen Kohlenwasserstoffs der Formel

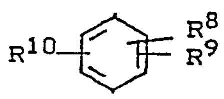

in der
$R^8$ bis $R^{10}$ unabhängig voneinander Wasserstoff, Alkyl oder Aryl bedeuten, wobei zwei der Reste $R^8$-$R^{10}$, wenn sie benachbart sind, einen anellierten aromatischen oder cycloaliphatischen Ring bilden können,
in Gegenwart von wasserfreiem Fe-(III)-halogenid bei 0-150° C umsetzt.

2. Verfahren nach Anspruch 1, dadurch gekennzeichnet, dass man 2-Benzyl-tert.-alkyltoluole der Formel

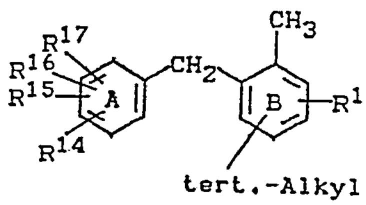

umsetzt, in der
$R^{11}$ und $R^{14}$ bis $R^{17}$ unabhängig voneinander Wasserstoff, Fluor, Chlor, Brom, Nitro, Alkyl oder Phenyl bedeuten, wobei je zwei der Reste $R^{14}$-$R^{17}$, wenn sie benachbart sind, einen anellierten aromatischen oder cycloaliphatischen Ring bilden können und die tert.-Alkyl-Gruppe in para-Stellung zur Methylgruppe oder in para-Stellung zur Methylengruppe steht.

3. Verfahren nach Ansprüchen 1 und 2, dadurch gekennzeichnet, dass man 2-Benzyl-tert.-Alkyl-toluole der Formel

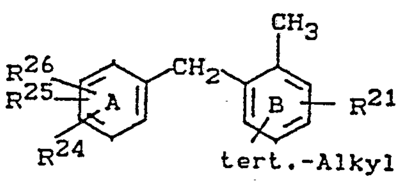

umsetzt, in der
$R^{21}$ Wasserstoff oder Alkyl bedeutet,
$R^{24}$, $R^{25}$ und $R^{26}$ unabhängig voneinander Wasserstoff, Fluor, Chlor, Brom, Nitro, Alkyl oder Phenyl bedeuten, wobei je zwei der genannten Reste, wenn sie benachbart sind, einen anellierten, aromatischen oder cycloaliphatischen Ring bilden können.

4. Verfahren nach Ansprüchen 1 bis 3, dadurch gekennzeichnet, dass man einen aromatischen Kohlenwasserstoff der Formel

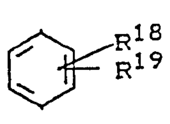

einsetzt, in der
$R^{18}$ und $R^{19}$ unabhängig voneinander Wasserstoff, Alkyl oder Aryl bedeuten und für den Fall, dass sie benachbart sind, auch einen anellierten aromatischen oder cycloaliphatischen Ring bilden können.

5. Verfahren nach Ansprüchen 1 bis 4, dadurch gekennzeichnet, dass man 0,1-1 Mol wasserfreies Eisen-(III)-halogenid pro Mal 2-Benzyl-tert.-alkyl-toluol einsetzt.

6. Verfahren nach Ansprüchen 1 bis 5, dadurch gekennzeichnet, dass man 2-20 Mol des aromatischen Kohlenwasserstoffs pro Mol 2-Benzyl-tert.-alkyl-toluol einsetzt.

7. Verfahren nach Ansprüchen 1 bis 6, dadurch gekennzeichnet, dass die Umsetzung bei 0-100° C durchgeführt wird.

8. Verfahren zur Herstellung von 2-Benzyltoluolen der Formel

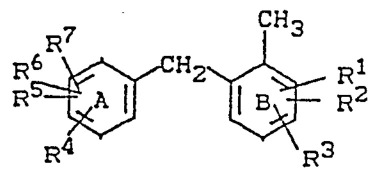

in der
$R^1$ bis $R^7$ unabhängig voneinander Wasserstoff, Halogen, Alkyl, Aryl, Nitro, Cyano, Acylamino, Aryloxy, Arylsulfonyl oder Alkylsulfonyl bedeuten, wobei im Kern B entweder die para-Position zur Methylgruppe oder die para-Position zur Methylengruppe unsubstituiert ist und wobei je zwei der Reste $R^1$-$R^7$, wenn sie benachbart sind, einen anellierten aromatischen oder cycloaliphatischen Ring bilden können,
dadurch gekennzeichnet, dass man in einer ersten Stufe Benzylhalogenide der Formel

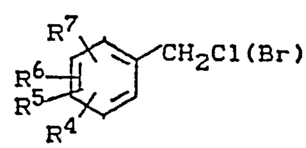

in der
$R^4$ bis $R^7$ die genannte Bedeutung haben, mit überschüssigem tert.-Alkyl-toluol der Formel

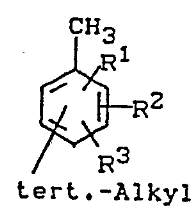

in der
$R^1$ bis $R^3$ die genannte Bedeutung haben, eine ortho-Position zur Methylgruppe frei ist und die tert.-Alkyl-Gruppe in para-Position zur Methylgruppe oder zur genannten freien ortho-Position steht,
bei erhöhter Temperatur in Gegenwart von wasserfreiem Eisen-(III)-halogenid umsetzt und in einer zweiten Stufe das erhaltene Reaktionsgemisch mit überschüssigem aromatischen Kohlenwasserstoff der Formel

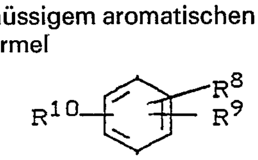

in der

$R^8$ bis $R^{10}$ unabhängig voneinander Wasserstoff, Alkyl oder Aryl bedeuten, wobei zwei der Reste $R^8$-$R^{10}$, wenn sie benachbart sind, einen anellierten aromatischen oder cycloaliphatischen Ring bilden können,

nach Anspruch 1 weiter umsetzt.

9. Verfahren nach Anspruch 8, dadurch gekennzeichnet, dass man vor Ausführung der zweiten Stufe den nicht umgesetzten Überschuss des tert.-Alkyl-toluols abdestilliert.

10. Verfahren nach Ansprüchen 8 und 9, dadurch gekennzeichnet, dass man als aromatischen Kohlenwasserstoff ein Toluol der Formel

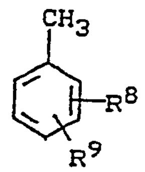

einsetzt, in der

$R^8$ und $R^9$ unabhängig voneinander Wasserstoff, Alkyl oder Aryl bedeuten und für den Fall, dass sie benachbart sind, auch einen anellierten aromatischen oder cycloaliphatischen Ring bilden können, und worin eine der ortho-Positionen zur Methylgruppe frei ist und die para-Position zur Methylgruppe oder zur genannten freien ortho-Position ebenfalls frei ist,

das im Verlaufe der Reaktion daraus entstehende tert.-Alkyl-toluol der Formel

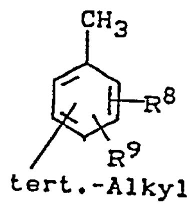

in der

$R^8$ und $R^9$ die genannte Bedeutung haben und worin eine der ortho-Positionen zur Methylgruppe frei ist und die tert.-Alkyl-Gruppe in para-Position zur Methylgruppe oder zur genannten freien ortho-Position steht,

isoliert und mit Benzylhalogeniden in der ersten Stufe erneut umsetzt.

## Claims

1. Process for the preparation of 2-benzyltoluenes of the formula

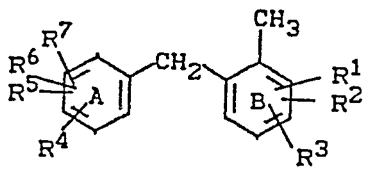

in which

$R^1$ to $R^7$, independently of one another, denote hydrogen, halogen, alkyl, aryl, nitro, cyano, acylamino, aryloxy, arylsulphonyl or alkylsulphonyl, where in nucleus B, either the para position to the methyl group or the para position to the methylene group is unsubstituted and where each two of the radicals $R^1$-$R^7$, when they are neighbouring, can form an anellated aromatic or cycloaliphatic ring, characterized in that 2-benzyl-tert.-alkyl-toluenes of the formula

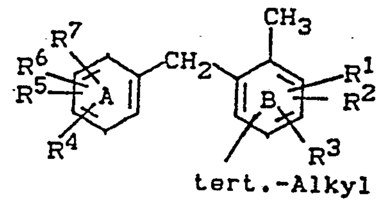

in which

$R^1$ to $R^7$ have the meaning mentioned and the tert.-alkyl group is in the para position to the methyl group or in the para position to the methylene group,

are reacted at 0-150° C with an excess of an aromatic hydrocarbon of the formula

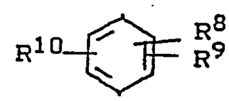

in which

$R^8$ to $R^{10}$, independently of one another, denote hydrogen, alkyl or aryl, where two of the radicals $R^8$-$R^{10}$, when they are neighbouring, can form an anellated aromatic or cycloaliphatic ring,

in the presence of anhydrous Fe-(III) halide.

2. Process according to Claim 1, characterized in that 2-benzyl-tert.-alkyl-toluenes of the formula

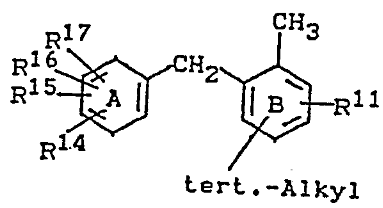

in which

$R^{11}$ and $R^{14}$ to $R^{17}$, independently of one another, denote hydrogen, fluorine, chlorine, bromine, nitro, alkyl or phenyl, where each two of the radicals $R^{14}$-$R^{17}$, when they are neighbouring, can form an anellated aromatic or cycloaliphatic ring and the tert.-alkyl group is in the para position to the methyl group or in the para position to the methylene group,

are reacted.

3. Process according to Claims 1 and 2, characterized in that 2-benzyl-tert.-alkyl-toluenes of the formula

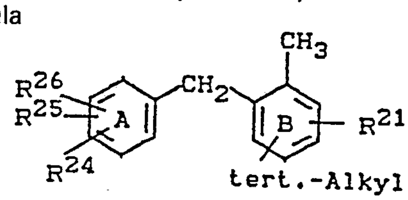

in which

$R^{21}$ denotes hydrogen or alkyl, and

$R^{24}$, $R^{25}$ and $R^{26}$, independently of one another, denote hydrogen, fluorine, chlorine, bromine, nitro, alkyl or phenyl, where each two of the radicals mentioned, when they are neighbouring, can form an anellated aromatic or cycloaliphatic ring, are reacted.

4. Process according to Claims 1 to 3, characterized in that an aromatic hydrocarbon of the formula

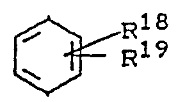

in which

$R^{18}$ and $R^{19}$, independently of one another, denote hydrogen, alkyl or aryl and, in the case where they are neighbouring, can also form an anellated aromatic or cycloaliphatic ring,

is employed.

5. Process according to Claims 1 to 4, characterized in that 0.1-1 mol of anhydrous iron-(III) halide is employed per mol of 2-benzyl-tert.-alkyltoluene.

6. Process according to Claims 1 to 5, characterized in that 2-20 mol of the aromatic hydrocarbon are employed per mol of 2-benzyl-tert.-alkyltoluene.

7. Process according to Claims 1 to 6, characterized in that the reaction is carried out at 0-100° C.

8. Process for the preparation of 2-benzyltoluenes of the formula

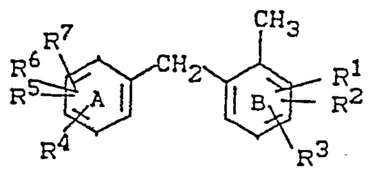

in which

$R^1$ to $R^7$, independently of one another, denote hydrogen, halogen, alkyl, aryl, nitro, cyano, acylamino, aryloxy, arylsulphonyl or alkylsulphonyl, where, in nucleus B, either the para position to the methyl group or the para position to the methylene group is unsubstituted and where each two of the radicals $R^1$-$R^7$, when they are neighbouring, can form an anellated aromatic or cycloaliphatic ring, characterized in that, in a first stage, benzyl halides of the formula

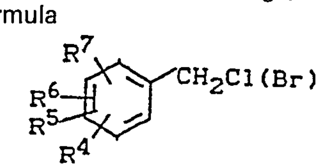

in which

$R^4$ to $R^7$ have the meaning mentioned,

are reacted with excess tert.-alkyl-toluene of the formula

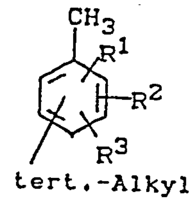

in which

$R^1$ to $R^3$ have the meaning mentioned, an ortho position to the methyl group is free and the tert.-alkyl group is in the para position to the methyl group or to the free ortho position mentioned, at elevated temperature in the presence of anhydrous iron-(III) halide, and, in a second stage, the reaction mixture obtained is further reacted, according to Claim 1, with excess aromatic hydrocarbon of the formula

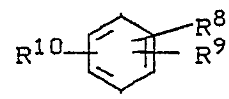

in which

$R^8$ to $R^{10}$, independently of one another, denote hydrogen, alkyl or aryl, where two of the radicals $R^8$-$R^{10}$, when they are neighbouring, can form an anellated aromatic or cycloaliphatic ring.

9. Process acording to Claim 8, characterized in that the unreacted excess of the tert.-alkyl-toluene is removed by distillation before carrying out the second stage.

10. Process according to Claims 8 and 9, characterized in that a toluene of the formula

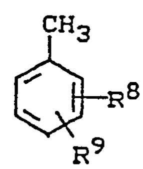

in which

$R^8$ and $R^9$, independently of one another, denote hydrogen, alkyl or aryl, and, in the case where they are neighbouring, can also form an anellated aromatic or cycloaliphatic ring, and in which one of ortho positions to the methyl group is free and the para position to the methyl group or to the free ortho position mentioned is likewise free,

is employed as aromatic hydrocarbon, and the tert.-alkyl-toluene of the formula

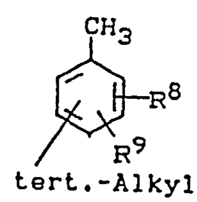

in which

$R^8$ and $R^9$ have the meaning mentioned and in which one of the ortho positions to the methyl group is free and the tert.-alkyl group is in the para position to the methyl group or to the free ortho position mentioned,

produced from this toluene in the course of the reaction is isolated and re-reacted, in the first stage, with benzyl halides.

**Revendications**

1. Procédé de préparation de 2-benzyltoluènes de formule

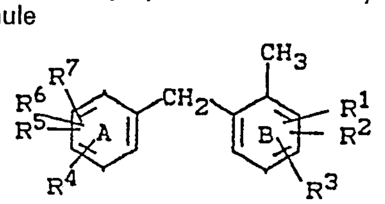

dans laquelle

$R^1$ à $R^7$ représentent, indépendamment l'un de l'autre, un atome d'hydrogène ou d'halogène, ou un groupe alkyle, aryle, nitro, cyano, acylamino, aryloxy, arylsulfonyle ou alkylsulfonyle, la position en para par rapport au groupe méthyle ou la position en para par rapport au groupe méthylène n'étant pas substituée sur le noyau B et deux des restes $R^1$ à $R^7$ pouvant, quand ils sont voisins, former un noyau aromatique ou cycloaliphatique condensé, procédé caractérisé en ce qu'on fait réagir entre 0 et 150° C, en présence d'un halogénure de Fe-(III) anhydre, des 2-benzyl-tertio-alkyltoluène de formule

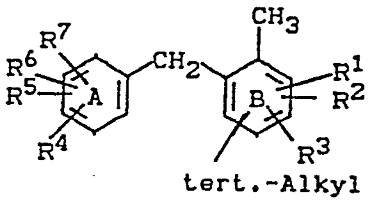

dans laquelle

$R^1$ à $R^7$ ont le sens précité et le groupe tertio-alkyle est en position para par rapport au groupe méthyle ou en position para par rapport au groupe méthylène,

avec un excès d'un hydrocarbure aromatique de formule:

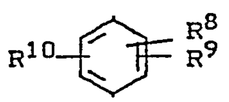

dans laquelle

$R^8$ à $R^{10}$ représentent, indépendamment l'un de l'autre, un atome d'hydrogène ou un groupe alkyle ou aryle, deux des restes $R^8$ à $R^{10}$ pouvant, quand ils sont voisins, former un noyau aromatique ou cycloaliphatique condensé.

2. Procédé selon la revendication 1, caractérisé en ce qu'on fait réagir des 2-benzyl-tertioalkyltoluènes de formule

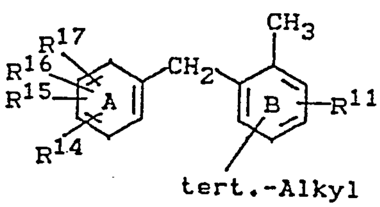

dans laquelle

$R^{11}$ et $R^{14}$ à $R^{17}$ représentent chacun, indépendamment l'un de l'autre, un atome d'hydrogène, de fluor, de chlore ou de brome ou un groupe nitro, alkyle ou phényle, deux des restes $R^{14}$ à $R^{17}$ pouvant, quand ils sont voisins, former un noyau aromatique ou cycloaliphatique condensé, et le groupe tertio-alkyle étant en position para par rapport au groupe méthyle ou en position para par rapport au groupe méthylène.

3. Procédé selon les revendications 1 et 2, caractérisé en ce qu'on fait réagir des 2-benzyl-tertio-alkyltoluènes de formule

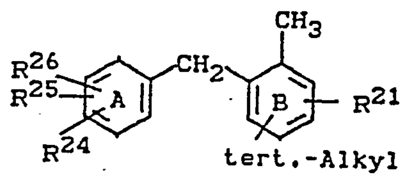

dans laquelle

$R^{21}$ représente un atome d'hydrogène ou un groupe alkyle,

$R^{24}$, $R^{25}$ et $R^{26}$ représentent, indépendamment l'un de l'autre, un atome d'hydrogène, de fluor, de chlore ou de brome, un groupe nitro, alkyle ou phényle, deux des restes cités pouvant, quand ils sont voisins, former un noyau aromatique ou cycloaliphatique condensé.

4. Procédé selon les revendications 1 à 3, caractérisé en ce qu'on utilise un hydrocarbure aromatique de formule

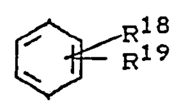

dans laquelle

$R^{18}$ et $R^{19}$ représentent, indépendamment l'un de l'autre, un atome d'hydrogène, un groupe alkyle ou aryle et, s'ils sont voisins, ils peuvent également former un noyau aromatique ou cycloaliphatique condensé.

5. Procédé selon les revendications 1 à 4, caractérisé en ce qu'on utilise 0,1 à 1 mole d'halogénure de fer-(III) anhydre par mole de 2-benzyl-tertio-alkyltoluène.

6. Procédé selon les revendications 1 à 5, caractérisé en ce qu'on utilise 2 à 20 moles de l'hydrocarbure aromatique par mole du 2-benzyl-tertio-alkyltoluène.

7. Procédé selon les revendications 1 à 6, caractérisé en ce qu'on conduit la réaction entre 0 et 100° C.

8. Procédé pour préparer des 2-benzyltoluènes de formule

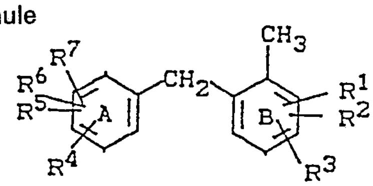

dans laquelle

$R^1$ à $R^7$ représentent, indépendamment l'un de l'autre, un atome d'hydrogène ou d'halogène, un groupe alkyle, aryle, nitro, cyano, acylamino, aryloxy, arylsulfonyle ou alkylsulfonyle, la position en para par rapport au groupe méthyle ou la position en para par rapport au groupe méthylène n'étant pas substituée dans le noyau B, et deux quelconques des restes $R^1$ à $R^7$ pouvant, quand ils sont voisins, former un noyau aromatique ou cycloaliphatique condensé, procédé caractérisé en ce que, en une première étape, on fait réagir à température élevée, en présence d'un halogénure de fer-(III), des halogénures de benzyle de formule

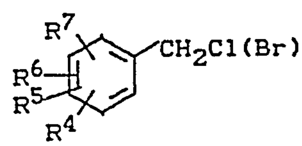

dans laquelle

$R^4$ à $R^7$ ont le sens précité,

avec un excès de tertio-alkyltoluène de formule

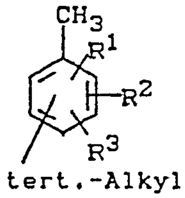

dans laquelle

$R^1$ à $R^3$ ont le sens précité, une position ortho par rapport au groupe méthyle étant libre et le groupe tertio-alkyle étant en position para par rapport au groupe méthyle ou par rapport à la position en ortho libre précitée,

et, dans une seconde étape, on fait réagir encore, selon la revendication 1, le mélange réactionnel ainsi obtenu avec un excès d'hydrocarbure aromatique de formule

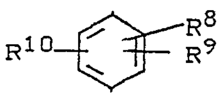

dans laquelle

$R^8$ à $R^{10}$ représentent, indépendamment l'un de l'autre, un atome d'hydrocarbure, un groupe alkyle ou aryle, deux des restes $R^8$ à $R^{10}$ pouvant, lorsqu'ils sont voisins, former un noyau aromatique ou cycloaliphatique condensé.

9. Procédé selon la revendication 8, caractérisé en ce que, avant la réalisation de la seconde étape, on chasse par distillation l'excès du tertio-alkyltoluène n'ayant pas réagi.

10. Procédé selon les revendications 8 et 9, caractérisé en ce qu'on utilise comme hydrocarbure aromatique un toluène de formule

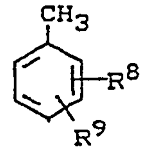

dans laquelle

$R^8$ et $R^9$ représentent, indépendamment l'un de l'autre, un atome d'hydrogène, un groupe alkyle ou aryle et, s'ils sont voisins, ils peuvent également former un noyau aromatique ou cycloaliphatique condensé et l'une des positions ortho par rapport au groupe méthyle est libre et la position en para par rapport au groupe méthyle ou par rapport à la position ortho citée comme libre est également libre;

on isole le tertio-alkyltoluène de formule

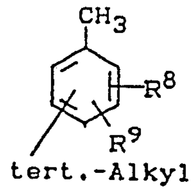

dans laquelle

$R^8$ et $R^9$ ont le sens précité et l'une des positions en ortho par rapport au groupe méthyle est libre et le groupe tertioalkyle est en position para par rapport au groupe méthyle ou par rapport à la position ortho libre précitée,

que l'on obtient au cours de la réaction, et on le fait réagir à nouveau avec des halogénures de benzyle dans la première étape.